(19) Europäisches Patentamt
European Patent Office
Office européen des brevets

(11) Publication number: **0 207 453 B1**

(12) # EUROPEAN PATENT SPECIFICATION

(45) Date of publication of patent specification: **22.06.94**

(51) Int. Cl.[5]: **C07D 413/12**, C07D 417/12, C07D 409/12, C07D 405/12, C07D 307/42, A61K 31/42, A61K 31/425, C07D 261/08, //C07D277/66,C07D277/24, C07D333/10

(21) Application number: **86108744.3**

(22) Date of filing: **26.06.86**

The file contains technical information submitted after the application was filed and not included in this specification

(54) **Heterocyclic substituted-phenoxyalkyl-isoxazoles and-furans, their preparation and use as antiviral agents.**

(30) Priority: **02.07.85 US 751348**

(43) Date of publication of application:
**07.01.87 Bulletin 87/02**

(45) Publication of the grant of the patent:
**22.06.94 Bulletin 94/25**

(84) Designated Contracting States:
**AT BE CH DE FR GB IT LI NL SE**

(56) References cited:
**EP-A- 0 111 345**
**EP-A- 0 137 242**

(73) Proprietor: **STERLING WINTHROP INC.**
**90 Park Avenue**
**New York, NY 10016(US)**

(72) Inventor: **Diana, Guy Dominic**
**Box 192**
**Garfield Road**
**East Nassau**
**Box 432 R.D.1, Nassau New York(US)**
Inventor: **Carabateas, Philip Michael**
**Box 192**
**Garfield Road**
**East Nassau**
**Box 432 R.D.1, Nassau New York(US)**

(74) Representative: **Baillie, Iain Cameron et al**
**c/o Ladas & Parry**
**Altheimer Eck 2**
**D-80331 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 207 453 B1

Rank Xerox (UK) Business Services
(3.10/3.09/3.3.3)

## Description

This invention relates to novel heterocyclic substituted-phenoxyalkylisoxazoles and -furans, to methods for the preparation thereof, and compositions and methods for the use thereof as antiviral agents.

Sterling Drug Inc. European Patent Application Publ. No. 111,345, published June 20, 1984 (U.S. Pat. 4,451,476, issued May 29, 1984) discloses antivirally active compounds having the formula

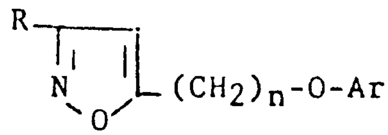

wherein:

R is alkyl of 1 to 3 carbon atoms;

n is an integer from 4 to 8; and

Ar is phenyl or phenyl substituted by one or two substituents selected from the group consisting of halogen, lower-alkyl, lower-alkoxy, nitro, cyano, carboxy, lower-alkoxycarbonyl, lower-alkanoyl, 1-oximino-lower-alkyl, hydrazinocarbonyl, carbamyl and N,N-di-lower-alkylcarbamyl.

Sterling Drug Inc. European Patent Application Publ. No. 137,242, published April 17, 1985, discloses antivirally active compounds having the formula

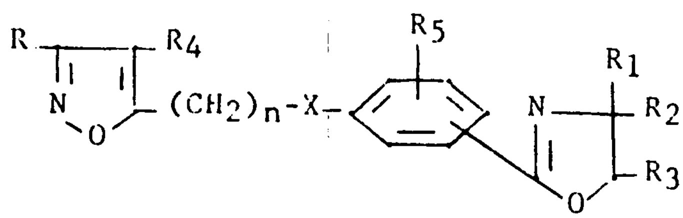

wherein:

R, $R_1$, $R_2$, $R_3$ and $R_4$ are each hydrogen or alkyl of 1 to 3 carbon atoms optionally substituted by hydroxy, lower-alkanoyloxy, lower-alkoxy, chloro, or N=Z, wherein N=Z is amino, lower-alkanoylamino, lower-alkylamino, di-lower-alkylamino, 1-pyrrolidinyl, 1-piperidinyl or 4-morpholinyl; with the proviso that R is other than hydrogen;

$R_5$ is hydrogen, lower-alkyl, halogen, nitro, lower-alkoxy, lower-alkylthio or trifluoromethyl;

X is O or a single bond; and

n is an integer from 3 to 9;

and to pharmaceutically acceptable acid-addition salts thereof.

It has now been found that compounds wherein the oxazoline ring of the compounds of the latter reference is replaced by selected other heterocycles are also effective antiviral agents.

Accordingly, the present invention relates to compounds of the formula

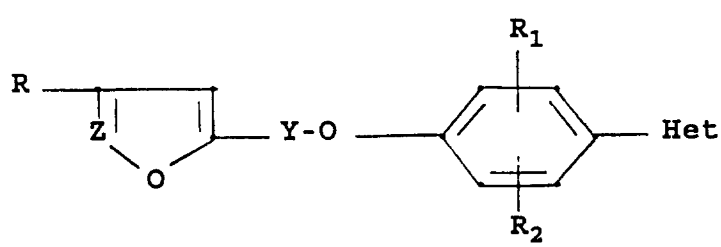

I

wherein:

Y is an alkylene bridge of 3-9 carbon atoms;

Z is N or HC;
R is hydrogen or lower-alkyl of 1-3 carbon atoms, with the proviso that when Z is N, R is lower-alkyl;
$R_1$ and $R_2$ are hydrogen, halogen, methyl, nitro, lower-alkoxycarbonyl or trifluoromethyl; and
Het is:

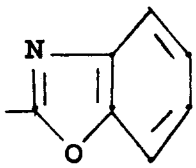 , 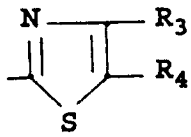 , 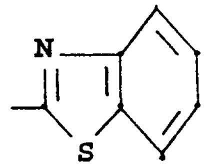 , 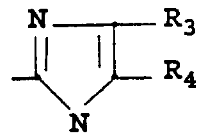 ,

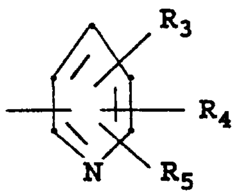 ,

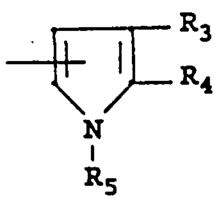 , 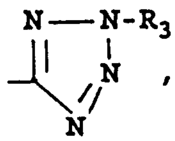 , 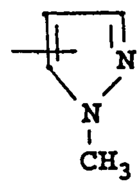

or 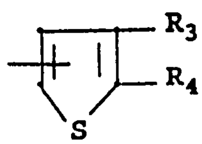 ; 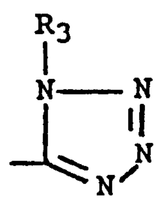 ,

and
$R_3$, $R_4$ and $R_5$ are hydrogen or lower-alkyl of 1-3 carbon atoms;
or pharmaceutically acceptable acid-addition salts of basic members thereof.

A preferred class of compounds within the scope of Formula I are those of the formula

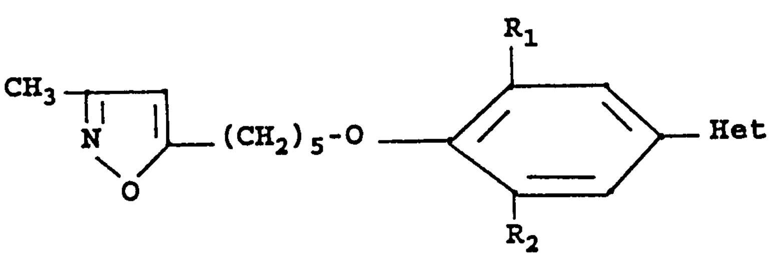

II

Compositions for combating viruses comprise an antivirally effective amount of a compound of Formulas **I** or **II** in admixture with a suitable carrier or diluent, and to methods of combating viruses therewith.

One can prepare a compound of Formula I by reacting a compound of the formula

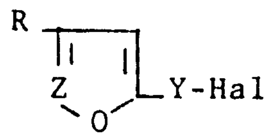

III

wherein Hal is chlorine, bromine or iodine, with an alkali metal salt of a compound of the formula

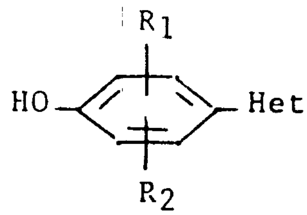

IV

One can also prepare a compound of Formula I by reacting a compound of the formula

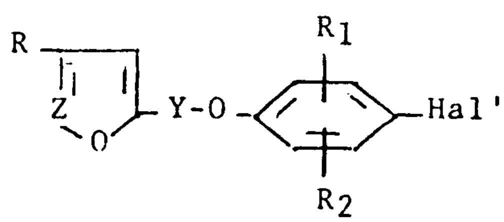

V

where Hal' is bromine or iodine, with a compound of the formula

$(R')_3$Sn-Het VI

where R' is lower-alkyl of 1-6 carbon atoms, and Het is as defined above; in the presence of a palladium complex catalyst.

Also disclosed are intermediates of the Formula V where R, Y and Z have the meanings given hereinabove; $R_1$ and $R_2$ are halogen, methyl, nitro, lower-alkoxycarbonyl or trifluoromethyl; and Hal' is bromine or iodine.

The compounds of Formula I where Het is a nitrogen-containing heterocyclic group are sufficiently basic to form stable acid-addition salts with strong acids, and said salts are within the purview of the invention. The nature of the acid-addition salt is immaterial, provided it is derived from an acid the anion of which is essentially non-toxic to animal organisms. Examples of appropriate acid-addition salts include the hydrochloride, hydrobromide, sulfate, acid sulfate, maleate, citrate, tartrate, methanesulfonate, p-toluenesulfonate, dodecyl sulfate, cyclohexanesulfamate, and the like.

When the term halogen is used to define the substituents $R_1$ and $R_2$, any of the four common halogens, fluorine, chlorine, bromine or iodine are contemplated; and the term lower-alkoxycarbonyl refers to such groups having from two to four carbon atoms.

The process for the preparation of compounds of Formula **I** by reacting intermediates of Formulas **III** and **IV** takes place by heating the reactants in an inert solvent in the presence of an alkali metal base, e.g.

potassium carbonate or potassium hydroxide at a temperature between about 50 °C and 150 °C.

The intermediates or Formula **III** where Z is N are prepared by reacting an alkali metal derivative of an isoxazole of the formula

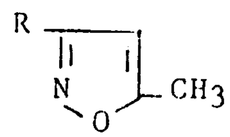

**VII**

with a dihalide, Hal-Y'-Hal, where Y' is an alkylene bridge of 2 to 8 carbon atoms. Said alkali metal derivative is prepared in situ by treating the compound of Formula **VII** with an organo-alkali metal base under anhydrous conditions. Preferred organo-alkali metal bases are butyllithium and lithium diisopropylamide.

The intermediates of Formula **III** where Z is HC are prepared from the appropriate omega-(2-furan)-alkanoic acid by reduction to the corresponding alcohol and replacement of the hydroxy group by halogen; or by direct alkylation of furan with a dihalide, Hal-Y-Hal, in the presence of a strong base such as butyllithium.

The intermediates of Formula **IV** are a generically known class of heterocyclic substituted phenols, prepared as described hereinafter in the general description and specific examples.

In the alternative process comprising reacting compounds of Formulas **V** and **VI**, the process is carried out using approximately equimolar amounts of the reactants in an inert solvent at a temperature between about 50° and 100 °C, conveniently at the reflux temperature of the solvent. The reaction is complete in a period ranging from 5-24 hours. The palladium complex catalyst, present to the extent of about 5 mole percent, can be any such catalyst known to effect cross-coupling of organotin compounds with organic halides [cf. Kosugi et al., Bull. Chem. Soc. Japan 59, 677-679 (1986)], for example $PdCl_2$-$(PPh_3)_2$, Pd-$(PPh_3)_4$, $PdCl_2[P(o\text{-tolyl})_3]_2$, $PdCl_2 + 2P(OEt)_3$ and $PdCl_2(PhCN)_2$. A preferred catalyst is dichlorobis-(triphenylphosphine)palladium $[PdCl_2(PPh_3)_2]$.

The intermediates of Formula **V** are prepared by reacting an alkali metal salt of a phenol of the formula

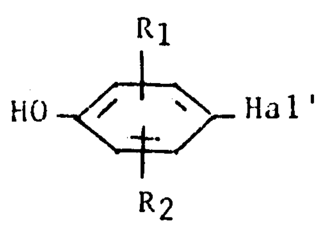

**VIII**

with a compound of Formula **III** in a procedure analogous to that of the reaction of **III** with **IV**.

The organotin reagent of Formula VI is prepared by known procedures comprising reacting a tri-lower-alkyl-tin halide with an unsubstituted aromatic heterocycle in the presence of a strong base such as butyllithium under anhydrous conditions. The trialkyltin moiety enters the most reactive position on the heterocyclic ring; however, the trialkyltin moiety can be directed to other positions on the heterocyclic ring by using the appropriate halo-substituted heterocycle.

Certain compounds of the invention can be prepared by construction of the Het ring from intermediates having a cyano or formyl group on the phenyl ring, as follows.

The compounds of Formula I where Het is a 2-thiazolyl group:

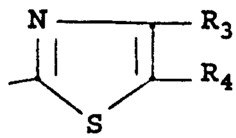

are prepared from the corresponding cyanophenyl compounds of the formula

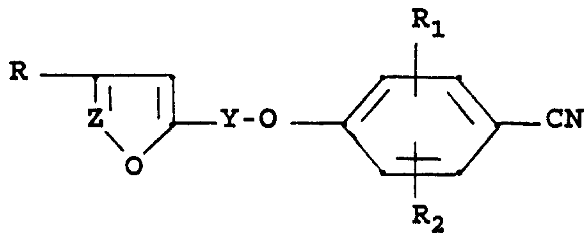

IX

by conversion of the latter to the corresponding thioamide with hydrogen sulfide in pyridine, and then reacting the thioamide with a haloalkanone, $R_3CH(Hal)-CO-R_4$.

The compounds of Formula I where Het is a tetrazole group:

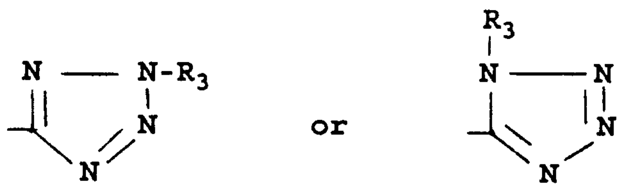

are prepared from the corresponding cyanophenyl compounds of Formula IX by reaction of the latter with sodium azide to give a tetrazole when $R_3$ is hydrogen. Treatment of the latter with a lower-alkyl halide in the presence of a base gives both isomeric tetrazoles where $R_3$ is lower-alkyl.

The structures of the compounds of the invention were established by the modes of synthesis, by elementary analysis, and by infrared and nuclear magnetic resonance spectra.

The following examples will further illustrate the invention.

Example 1

a) 2,6-Dichloro-4-iodophenyl trimethylsilyl ether.

A mixture of 5.25 g 2,6-dichloro-4-iodophenol and 2.0 ml (1 equiv.) di(trimethylsilyl)amine was heated at reflux for 3 hrs. The resulting trimethyl silyl ether, obtained in essentially quantitative yield, was used in the next reaction without further purification.

b) 2,6-Dichloro-4-(2-thienyl)phenol [**IV**; $R_1$ and $R_2$ = Cl, Het = 2-thienyl].

To a solution of 1.7 g thiophene in 20 ml ether at 0°C under nitrogen was added 1.9 ml 10.5M n-butyllithium. The mixture was kept at room temperature for 1 hr and then cooled to -20°C. Cuprous iodide (3.8 g) was then added, and the mixture was allowed to warm to 0°C and the solvent removed in vacuo. To the residue was added 20 ml dry pyridine and 7.2 ml 2,6-dichloro-4-iodophenyl tri-methylsilyl ether. The mixture was heated at reflux for 3.5 hrs and the product isolated and purified by flash filtration (5:1 hexane:ethyl acetate, silica gel) to give 2.3 g (47%) 2,6-dichloro-4-(2-thienyl)phenol as a yellow solid.

c) 5-{5-[2,6-Dichloro-4-(2-thienyl)phenoxy]pentyl}-3-methylisoxazole [**II**; $R_1$ and $R_2$ = Cl, Het = 2-thienyl].

A solution of 2.3 g 2,6-dichloro-4-(2-thienyl)-phenol, 2.2 g 5-(5-bromopentyl)-3-methylisoxazole and 0.7 g potassium hydroxide in 50 ml acetonitrile was heated at reflux for 5 hrs. Filtration, concentration and flash chromatography (4:1 hexane:ethyl acetate) provided 4.3 g of a light yellow oil which was

recrystallized from isopropyl acetate-hexane to give 1.8 g (49%) 5-{5-[2,6-dichloro-4-(2-thienyl)phenoxy]-pentyl}-3-methylisoxazole, pale-yellow solid, m.p. 45-47 °C upon further recrystallization from isopropyl acetate-hexane.

The intermediate 5-(5-bromopentyl)-3-methylisoxazole was prepared according to known methods from 1,4-dibromobutane and the lithium salt of 3,5-dimethylisoxazole produced in situ with n-butyllithium and diisopropylamine in tetrahydrofuran solution.

It is contemplated that the 5-(5-bromopentyl)-3-methylisoxazole can be replaced by the homologous 5-(3-bromopropyl)-3-methylisoxazole, 5-(7-bromoheptyl)-3-methylisoxazole or 5-(9-bromononyl)-3-methylisoxazole to give respectively 5-{3-[2,6-dichloro-4-(2-thienyl)phenoxy]propyl}-3-methylisoxazole [**I**; Y = $(CH_2)_3$, Z = N, R = $CH_3$, $R_1$ = 2-Cl, $R_2$ = 6-Cl, Het = 2-thienyl], 5-{7-[2,6-dichloro-4-(2-thienyl)-phenoxy]heptyl}-3-methylisoxazole [**I**; Y = $(CH_2)_7$, Z = N, R = $CH_3$, $R_1$ = 2-Cl, $R_2$ = 6-Cl, Het = 2-thienyl], or 5-{9-[2,6-dichloro-4-(2-thienyl)phenoxy]-nonyl}-3-methylisoxazole [**I**; Y = $(CH_2)_9$, Z = N, R = $CH_3$, $R_1$ = 2-Cl, $R_2$ = 5-Cl, Het = 2-thienyl].

It is further contemplated that 2,6-dichloro-4-(2-thienyl)phenol can be caused to react with 2-(5-bromopentyl)furan (prepared from furan and 1,5-dibromopentane) in accordance with the procedure of Example 1(c) to give 2-{5-[3,5-dichloro-4-(2-thienyl) phenoxy]pentyl}furan [I; Y = $(CH_2)_5$, Z = HC, R = H, $R_1$ = 2-Cl, $R_2$ = 5-Cl, Het = 2-thienyl].

Example 2

a) 4-(2-Thienyl)phenol [IV; $R_1$ and $R_2$ = H, Het = 2-thienyl].

To a solution of 8.80 g 2-(4-methoxyphenyl)-thiophene (prepared from 2-thienylmagnesium bromide and p-iodoanisole) and 5.4 ml propanethiol in 100 ml dry dimethylformamide was carefully added 7.00 g 35% potassium hydride in mineral oil. The reaction mixture was heated at reflux under nitrogen for 16 hours. Isolation of the resulting product afforded after crystallization 2.42 g 4-(2-thienyl)phenol as a yellow powder.

b) 3-Methyl-5-{5-(4-(2-thienyl)phenoxy]pentyl}-isoxazole [II; $R_1$ and $R_2$ = H, Het = 2-thienyl] was prepared from 2.42 g 4-(2-thienyl)phenol and 5-(5-bromopentyl)-3-methylisoxazole according to the procedure of Example 4, and was obtained in 38% yield (1.7 g) as an off-white solid, m.p. 105-107 °C. (from isopropyl acetate-hexane).

Example 3

a) 2-(4-Methoxyphenyl)-4,5-dimethylthiazole.

To a stirred mixture of 65.1 g (4-methoxy) thiobenzamide and 156 ml ethanol was added dropwise 50.1 g 3-chloro-2-butanone, and the reaction mixture was heated at reflux for 3 hours. An additional 6.1 g 2-chloro-3-butanone was added and heating was continued for an additional hour. The reaction mixture was cooled, 300 ml ether added, and the solid which precipitated was collected and dried to give 74.5 g 2-(4-methoxyphenyl)-4,5-dimethylthiazole in the form of its hydrochloride monohydrate, m.p. 166-170 °C.

b) 4-(4,5-Dimethyl-2-thiazolyl)phenol [IV; $R_1$ and $R_2$ = H, Het = (4,5-dimethyl-2-thiazolyl)].

The product of part (a) (70.7 g) was added to 470 g pyridine saturated with hydrogen chloride gas, and the mixture was heated 2 hours at reflux. The reaction mixture was poured into 3000 ml ice-water, made basic with ammonium hydroxide, and the solid product was collected. The latter was purified by recrystallization from toluene to give 38.8 g 4-(4,5-dimethyl-2-thiazolyl)phenol, m.p. 194-195 °C.

c) 3-Methyl-5-{5-[4-(4,5-dimethyl-2-thiazolyl)phenoxy]-pentyl}isoxazole [II; $R_1$ and $R_2$ = H, Het = (4,5-dimethyl-2-thiazolyl)] was prepared from 2.0 g 4-(4,5-dimethyl-2-thiazolyl)phenol and 2.3 g 5-(5-bromopentyl)-3-methylisoxazole according to the procedure of Example 4: yield 2.5 g, m.p. 96-97 °C. (yellow to orange crystals from ethyl acetate).

Example 4

a) 2-(4-Hydroxyphenyl)benzothiazole [IV; $R_1$ and $R_2$ = H, Het = benzothiazol-2-yl].

A mixture of 3.7 g 2-aminothiophenol, 4.2 g 4-hydroxybenzoic acid, 4.5 g phosphorus pentoxide and 45 g methanesulfonic acid was stirred for one hour at room temperature and then heated at 90 °C. for 10 hours. The reaction mixture was poured slowly into 750 ml 5% sodium bicarbonate solution. The solid which precipitated was collected and dried to give 7.0 g 2-(4-hydroxyphenyl)-benzothiazole.

b) 2-{4-[[5-(3-Methyl-5-isoxazolyl)pentyl]oxy]phenyl}-benzothiazole [II; $R_1$ and $R_2$ = H, Het = benzothiazol-2-yl] was prepared from 5 g 2-(4-hydroxyphenyl)-benzothiazole and 5.1 g 5-(5-bromopentyl)-3-methylisoxazole according to the procedure of Example 6: yield 6.9 g, m.p. 120-121 °C. when recrystallized from triethylamine and then from isopropyl acetate.

Example 5

a) 4-(2-Benzothiazolyl)-2-nitrophenol [IV; $R_1$ = $NO_2$, $R_2$ = H, Het = benzothiazol-2-yl].

A mixture of 14.8 g 2-aminothiophenol, 21.6 g 4-hydroxy-3-nitrobenzoic acid, 18 g phosphorus pentoxide and 180 g methanesulfonic acid was heated at 90 °C. for 10 hours. The reaction mixture was brought to pH 5 by addition of sodium bicarbonate and sodium hydroxide solutions, and the solid product collected. The latter was recrystallized first from ethyl acetate and then from acetonitrile to give 6.5 g of the above-indicated product, orange-brown needles, m.p. 214-215 °C.

b) 5-{5-[4-(2-Benzothiazolyl)-2-nitrophenoxy]pentyl}-3-methylisoxazole [II; $R_1$ = $NO_2$, $R_2$ = H, Het = benzothiazol-2-yl] can be prepared by reacting 4-(2-benzothiazolyl)-2-nitrophenol with 5-(5-bromopentyl)-3-methylisoxazole in accordance with the procedure of Example 6.

Example 6

5-{5-[4-(2-Benzoxazolyl)phenoxy]pentyl}-3-methylisoxazole [II; $R_1$ and $R_2$ = H, Het = benzoxazol-2-yl] was prepared from 5 g 2-(4-hydroxyphenyl)-benzoxazole (prepared by heating 4-hydroxybenzamide with 2-aminophenol) and 11.1 g 5-(5-bromopentyl)-3-methylisoxazole and 40 g milled potassium carbonate in 1.5 liters acetonitrile under nitrogen. The mixture was heated to reflux. A catalytic amount of sodium iodide was added and refluxing continued for 4 hrs. The reaction mixture was filtered and concentrated to a solid residue. The latter was dissolved in ethyl acetate and the solution washed with water and saturated sodium chloride solution, dried over magnesium sulfate and concentrated in vacuo. There was obtained 5.35 g of the above-indicated product, m.p. 96-98 °C. (from isopropyl acetate).

General Technique A for Production of Organotin Reactants

To a solution of the desired heterocyclic compound in dry ether at 0 °C. under nitrogen was added n-butyllithium. The reaction mixture was heated at reflux for 15 min., then cooled to -30 °C. and trimethyltin chloride was added. The reaction mixture was allowed to warm to room temperature and then poured into water. The ether layer was separated, washed with water and passed through an alumina column, eluting with hexane.

General Technique B for Preparation of 5-[5-(2,6-dimethyl-4-iodophenoxy)pentyl]-3-methylisoxazole

[V; Y = $(CH_2)_5$, Z = N, R = $CH_3$, $R_1$ = 2-$CH_3$, $R_2$ = 6-$CH_3$, Hal' = I] was prepared from 10.0 g 2,6-dimethyl-4-iodophenol, 9.4 g 5-(5-bromopentyl)-3-methylisoxazole and 2.9 g potassium hydroxide in 100 ml acetonitrile by a procedure analogous to that of Example 1(c), and was obtained as a pale-yellow oil (15.0 g, 94%) after chromatography.

General Technique C for Reaction of Products of General Techniques A and B

A mixture of 5-[5-(2,6-dimethyl-4-iodophenoxy)pentyl]-3-methylisoxazole produced according to General Technique A, organotin reactant produced according to General Technique B, and dichlorobis-(triphenylphosphine)palladium in tertrahydrofuran was heated at reflux under nitrogen for 5 hrs. The reaction mixture was extracted with ether and the ether extracts washed with water and chromatographed.

General Technique D

To a solution of the desired heterocyclic compound in 100 ml ether at -78 °C. under nitrogen was added dropwise 9.5M n-butyllithium. There was then added a solution of trimethyltin chloride in 15 ml ether cooled to -78 °C. The reaction mixture after warming to room temperature was filtered and extracted with ether. The ether extracts were dried over potassium carbonate, concentrated, and passed through neutral alumina.

Example 7

a) 2-(Tributylstannyl)thiophene [VI; R' = $(CH_2)_3CH_3$, Het = 2-thienyl] was prepared from thiophene and tri(n-butyl)tin chloride according to the above described General Technique A for Preparing Organotin Reactants, and was obtained in essentially quantitative yield as a pale-yellow oil.

b) 5-{5-[2,6-Dimethyl-4-(2-thienyl)phenoxy]pentyl}-3-methylisoxazole [II; $R_1$ and $R_2$ = $CH_3$, Het = 2-thienyl] was prepared from 5-[5-(2,6-dimethyl-4-iodophenoxy)pentyl]-3-methylisoxazole (General Technique B) and 2-(tributylstannyl)thiophene according to the procedure of General Technique C and was obtained in 57% yield as a pale-yellow solid, m.p. 45-47 °C. (from isopropyl acetate-hexane).

Example 8

5-{5-[2,6-Dichloro-4-(2-thienyl)phenoxy]pentyl}-3-methylisoxazole [II; $R_1$ and $R_2$ = Cl, Het = 2-thienyl] was prepared from 5-[5-(2,6-dichloro-4-iodophenoxy) pentyl]-3-methylisoxazole and 2-(tributylstannyl)thiophene (Example 7a) according to the procedure of General Technique C, and was obtained in 76% yield as a colorless solid, m.p. 50-52 °C. (from isopropyl acetate-hexane). This compound is identical with the compound of Example 1(c).

Example 9

a) 5-Methyl-2-(trimethylstannyl)thiophene [VI; R' = $CH_3$, Het' = 5-methyl-2-thienyl] was prepared from 2-methylthiophene and trimethyltin chloride according to the procedure of General Technique A and was obtained in 89% yield as a pale-yellow oil, $n_D^{22}$ = 1.5385.

b) 5-{5-[2,6-Dimethyl-4-(5-methyl-2-thienyl)phenoxy] pentyl}-3-methylisoxazole [II; $R_1$ and $R_2$ = $CH_3$, Het = 5-methyl-2-thienyl] was prepared from 5-[5-(2,6-dimethyl-4-iodophenoxy)pentyl]-3-methylisoxazole and 5-methyl-2-(trimethylstannyl)thiophene according to the procedure of General Technique C, and was obtained in about 40% yield as an off-white solid, m.p. 64.5-65.5 °C. (from hexane).

Example 10

a) 5-Methyl-2-(tributylstannyl)thiophene [VI; R' = $(CH_2)_3CH_3$, Het = 5-methyl-2-thienyl] was prepared from 2-methylthiophene and tri(n-butyl)tin chloride according to the procedure of General Technique A, and was obtained in 97% yield as a colorless liquid.

b) 5-{5-[2,6-Dichloro-4-(5-methyl-2-thienyl)phenoxy] pentyl}-3-methylisoxazole [II; $R_1$ and $R_2$ = Cl, Het = 5-methyl-2-thienyl] was prepared from 5-[5-(2,6-dichloro-4-iodophenoxy)pentyl]-3-methylisoxazole and 5-methyl-2-(tributylstannyl)thiophene according to the procedure of General Technique C, and was obtained in about 45% yield as a colorless solid, m.p. 43-45 °C. (from isopropyl acetate-hexane).

Example 11

a) 3-(Trimethylstannyl)thiophene [VI; R' = $CH_3$, Het = 3-thienyl] was prepared from 3-bromothiophene and trimethyltin chloride according to the procedure of General Technique D, and was obtained in 93% yield as a pale-yellow liquid.

b) 5-{5-[2,6-Dimethyl-4-(3-thienyl)phenoxy]pentyl}-3-methylisoxazole [II; $R_1$ and $R_2$ = $CH_3$, Het = 3-thienyl] was prepared from 5-[5-(2,6-dimethyl-4-iodophenoxy)pentyl]-3-methylisoxazole and 3-(trimethylstannyl)thiophene according to the procedure of General Technique C, and was obtained in 47% yield as a colorless solid, m.p. 53-54 °C. (from isopropyl acetate-hexane).

Example 12

5-{5-[2,6-Dichloro-4-(3-thienyl)phenoxy]pentyl}-3-methylisoxazole [II; $R_1$ and $R_2$ = Cl, Het = 2-thienyl] was prepared from 5-[5-(2,6-dichloro-4-iodophenoxy)pentyl]-3-methylisoxazole and 3-(trimethylstannyl)thiophene (Example 11a) according to the procedure of General Technique C, and was obtained in 84% yield as a colorless solid, m.p. 43-44 °C. (from isopropyl acetate-hexane).

Example 13

a) 1-Methyl-2-(trimethylstannyl)pyrrole [VI; R' = $CH_3$, Het' = 1-methyl-2-pyrrolyl] was prepared from 1-methylpyrrole and trimethyltin chloride according to the procedure of General Technique A, and was obtained in 76% yield as a light orange oil.

b) 5-{5-[2,6-Dimethyl-4-(1-methyl-1H-pyrrol-2-yl)phenoxy]pentyl}-3-methylisoxazole [II; $R_1$ and $R_2$ = $CH_3$, Het = 1-methylpyrrol-2-yl] was prepared from 5-[5-(2,6-dimethyl-4-iodophenoxy)pentyl]-3-methylisoxazole and 1-methyl-2-(trimethylstannyl)pyrrole according to the procedure of General Technique C, and was obtained in 25% yield as a clear, viscous amber oil.

Example 14

5-{5-[2,6-Dichloro-4-(1-methyl-1H-pyrrol-2-yl)phenoxy]-pentyl}-3-methylisoxazole [II; $R_1$ and $R_2$ = Cl, Het = 1-methylpyrrol-2-yl] was prepared from 5-[5-(2,6-dichloro-4-iodophenoxy)pentyl]-3-methylisoxazole and 1-methyl-2-(trimethylstannyl)pyrrole (Example 13a), according to the procedure of General Technique C, and was obtained in 89% yield as a colorless solid, m.p. 46-47 ° C. (from isopropyl acetate-hexane).

Example 15

a) 2-(Trimethylstannyl)pyridine [VI; R' = $CH_3$, Het = 2-pyridinyl] was prepared from 2-bromopyridine and trimethyltin chloride according to the procedure of General Technique D, and was obtained in 95% crude yield as an orange liquid, $n_D^{23}$ = 1.5310. Fractionation provided a product with b.p. 74 ° C. (4.5 mm),

$$n_D^{22.5} = 1.5356.$$

b) 5-{5-[2,6-Dimethyl-4-(2-pyridinyl)phenoxyl]pentyl}-3-methylisoxazole [II; $R_1$ and $R_2$ = $CH_3$, Het = 2-pyridinyl] was prepared from 5-[5-(2,6-dimethyl-4-iodophenoxy)pentyl]-3-methylisoxazole and 2-(trimethylstannyl)pyridine according to the procedure of General Technique C, and was obtained in about 35% yield as a colorless solid, m.p. 31.5-32.5 ° C. (from ether-pentane).

Example 16

5-{5-[2,6-Dichloro-4-(2-pyridinyl)phenoxy]pentyl}-3-methylisoxazole [II; $R_1$ and $R_2$ = Cl, Het = 2-pyridinyl] was prepared from 5-[5-(2,6-dichloro-4-iodophenoxy) pentyl]-3-methylisoxazole and 2-(trimethylstannyl)-pyridine (Example 15a), according to the procedure of General Technique C, and was obtained in 80% yield as a colorless solid, m.p. 70.5-71.25 ° C. (from hexane).

Example 17

a) 3-(Trimethylstannyl)pyridine [VI; R' = $CH_3$, Het = 3-pyridinyl] was prepared from 3-bromopyridine and trimethyltin chloride according to the procedure of Example 15(a), and was obtained in 93% crude yield, $n_D^{22}$ = 1.5393 after fractionation.

b) 5-{5-[2,6-Dichloro-4-(3-pyridinyl)phenoxy]pentyl}-2-methylisoxazole [II; $R_1$ and $R_2$ = Cl, Het = 3-pyridinyl] was prepared from 5-[5-(2,6-dichloro-4-iodophenoxy)pentyl]-3-methylisoxazole and 3-(trimethylstannyl)pyridine, according to the procedure of General Technique C, and was obtained in about 30% yield as colorless needles, m.p. 84-84.8 ° C. (from hexane).

Example 18

a) 4-(Trimethylstannyl)pyridine [VI; R' = $CH_3$, Het = 4-pyridinyl] was prepared from 4-bromopyridine and trimethyltin chloride according to the procedure of General Technique D, and was obtained in 95% crude yield as a liquid, $n_D^{22}$ = 1.5346. Fractionation at 41 ° C. (0.06 mm) afforded material with $n_D^{22}$ = 1.5413.

b) 5-{5-[2,6-Dimethyl-4-(4-pyridinyl)phenoxy]-penty}-2-methylisoxazole [II; $R_1$ and $R_2$ = $CH_3$, Het = 4-pyridinyl] was prepared from 5-[5-(2,6-dimethyl-4-iodophenoxy)pentyl]-3-methylisoxazole and 4-(trimethylstannyl)pyridine, according to the procedure of General Technique C, and was obtained in about 65% yield as a colorless solid, m.p. 65-66 ° C. (from hexane).

Example 19

5-{5-[2,6-Dichloro-4-(4-pyridinyl)phenoxy]pentyl}-2-methylisoxazole [II; $R_1$ and $R_2$ = Cl, Het = 4-pyridinyl] was prepared from 5-[5-(2,6-dichloro-4-iodophenoxy)-pentyl]-3-methylisoxazole and 4-(trimethylstannyl)-pyridine (Example 18a), according to the procedure of General Technique C, and was obtained in 42% yield as an off-white solid, m.p. 79.5-80 ° C. (from ether).

By analogous procedures it is contemplated that benzoxazole and benzothiazole can be converted to the respective 2-(trimethylstannyl) derivatives, and the latter caused to react with 5-[5-(2,6-dichloro-4-iodophenoxy)pentyl]-3-methylisoxazole to give, respectively, 5-{5-[2,6-dichloro-4-(benzoxazol-2-yl)phenoxy]-pentyl}-3-methylisoxazole [II; $R_1$ and $R_2$ = Cl, Het = benzoxazol-2-yl] and 5-{5-[2,6-dichloro-4-(benzothiazol-2-yl)phenoxyl]pentyl}-3-methylisoxazole [II; $R_1$ and $R_2$ = Cl, Het = benzothiazol-2-yl].

Example 20

a) 5-[5-(4-Thiocarbamylphenoxy)pentyl]-3-methylisoxazole.

Hydrogen sulfide gas was bubbled through a solution of 17.49 g 5-[5-(4-cyanophenoxy)pentyl]-3-methylisoxazole and 4.5 ml triethylamine in 105 ml pyridine for a two hour period. The reaction mixture was allowed to stand for 20 hours and the solvent was removed in vacuo. The residue was recrystallized from acetonitrile to give 10.2 g 5-[5-(4-thiocarbamylphenoxy)pentyl]-3-methylisoxazole as a yellow solid, m.p. 156-158 ° C.

b) 3-Methyl-5-{5-[4-(2-thiazolyl)phenoxy]pentyl}-isoxazole [I; Z = N, Het = 4-(2-thiazolyl), R = $CH_3$, $R_1$ and $R_2$ = H, Y = $(CH_2)_5$].

A suspension of 7.5 g 5-[5-(4-thiocarbamylphenoxy)pentyl)-3-methylisoxazole and 24.6 g chloroacetaldehyde (50% in water) in 150 ml absolute ethanol was refluxed for 3.5 hours. The reaction mixture was concentrated in vacuo and the residue recrystallized from ethyl acetate-hexane to give 4.5 g 3-methyl-5-{5-[4-(2-thiazolyl)phenoxy]pentyl}isoxazole, m.p. 78-80 ° C.

Example 21

a) 5-[5-(2,6-Dichloro-4-cyanophenoxy)pentyl]-3-methylisoxazole [IX; Y = $(CH_2)_5$, Z = N, R = $CH_3$, $R_1$ and $R_2$ = Cl] was prepared from 13.4 g 3,5-dichloro-4-hydroxybenzonitrile, 23.2 g 5-(5-bromopentyl)-3-methylisoxazole, 20.7 g potassium carbonate and 15 g sodium iodide in 250 ml dimethylformamide, and was obtained in the form of colorless crystals (11.6 g), m.p. 59-60 ° C. (from tertiary-butyl methyl ether-hexane).

b) 5-[5-(2,6-Dichloro-4-carbamylphenoxy)pentyl]-3-methylisoxazole was prepared from 7.3 g 5-[5-(2,6-dichloro-4-cyanophenoxy)pentyl]-3-methylisoxazole, hydrogen sulfide and 2 ml triethylamine in 100 ml pyridine according to the procedure of Example 20(a), and was obtained in the form of a yellow solid (8.0 g), m.p. 138-140 ° C.

c) 5-{5-[2,6-Dichloro-4-(4,5-dimethyl-2-thiazolyl)-phenoxy]pentyl}-3-methylisoxazole [II; $R_1$ and $R_2$ = Cl, Het = 4,5-dimethyl-2-thiazolyl].

A mixture of 3.6 g 5-[5-(2,6-dichloro-4-carbamylphenoxy)pentyl]-3-methylisoxazole and 3.0 g 3-bromo-2-butanone in 50 ml absolute ethanol was heated at reflux for 6 hrs. The reaction mixture was concentrated in vacuo and the residue partitioned between ether and saturated sodium bicarbonate solution. From the ether layer was isolated an orange oil which after chromatography and recrystallization from ether-hexane gave 5-{5-[2,6-dichloro-4-(4,5-dimethyl-2-thiazolyl)phenoxy]pentyl}-3-methylisoxazole as a colorless solid, m.p. 57-58 ° C.

Example 22

5-{5-[2,6-Dichloro-4-(1H-tetrazol-5-yl)phenoxy]pentyl}-3-methylisoxazole [II; $R_1$ and $R_2$ = Cl, Het = 1H-tetrazol-5-yl].

A mixture of 14.7 g 5-[5-(2,6-dichloro-4-cyanophenoxy)pentyl]-3-methylisoxazole (Example 21a), 2.96 g sodium azide and 0.32 g ammonium chloride in 150 ml dimethylformamide was stirred and heated at 100 ° C. for 24 hrs. The reaction mixture was concentrated in vacuo, and the residue was dissolved in ethyl acetate and washed with 2N hydrochloric acid. The organic layer was dried over magnesium sulfate and

concentrated in vacuo to a solid residue. The latter was recrystallized from ethyl acetate to give 10.3 g (62%) 5-{5-[2,6-dichloro-4-(1H-tetrazol-5-yl)phenoxy]pentyl}-3-methylisoxazole, m.p. 122-123 ° C.

Example 23

A mixture of 6.4 g 5-{5-[2,6-dichloro-4-(1H-tetrazol-5-yl)phenoxy]pentyl}-3-methylisoxazole (Example 22), 2.8 g methyl iodide and 3.0 g milled potassium carbonate in 200 ml acetonitrile was stirred at room temperature overnight. The reaction mixture was filtered and concentrated in vacuo. The residue was dissolved in ethyl acetate and the solution washed with water, saturated sodium chloride solution, dried over magnesium sulfate and concentrated in vacuo. The residual oil was chromatographed using a hexane-ethyl acetate solvent system. Hexane:ethyl acetate 2:1 eluant brought out a first product which when recrystallized from ether-hexane gave 4.24 g (64%) of 5-{5-[2,6-dichloro-4-(2-methyl-2H-tetrazol-5-yl)phenoxy]-pentyl}-3-methylisoxazole [II; $R_1$ and $R_2$ = Cl, Het = 2-methyl-2H-tetrazol-5-yl], colorless solid, m.p. 47-49 ° C. Hexane:ethyl acetate 3:2 eluant brought out a second product which when recrystallized from ethyl acetate-hexane gave 890 mg (13%) of 5-{5-[2,6-dichloro-4-(1-methyl-1H-tetrazol-5-yl)phenoxy]pentyl}-3-methylisoxazole [II; $R_1$ and $R_2$ = Cl, Het = 1-methyl-1H-tetrazol-5-yl], colorless solid, m.p. 75-76 ° C.

Biological evaluation of compounds of Formulas I and II has shown that they possess antiviral activity. They are useful in inhibiting virus replication in vitro and are primarily active against picornaviruses, including polioviruses and especially numerous strains of rhinoviruses. The in vitro testing of the compounds of the invention against picornaviruses showed that viral replication was inhibited at minimum inhibitory concentrations (MIC) ranging from about 0.01 to about 5 micrograms per milliliter.

The MIC values were determined by a standard plaque reduction assay as follows: HeLa (Ohio) cells in monolayers were infected at a concentration of virus to give approximately 80 plaques per monolayer in the virus control (no drug present). The compound to be tested was serially diluted and included in the agar-medium overlay and in some cases, during the adsorption period as well. The MIC was determined to be that concentration of compound which reduced the number of plaques by 50% with respect to the untreated virus control.

In the standard test procedure, the compounds were tested against a panel of fifteen human rhinovirus (HRV) serotypes, namely HRV-2, -1A, -1B, -6, -14, -21, -22, -15, -25, -30, -50, -67, -89, -86 and -41. The MIC value for each rhinovirus serotype was determined, and the efficacy of each compound was determined in terms of a $MIC_{80}$ value, which is the concentration of the compound required to inhibit 80% of the tested serotypes.

The following Table gives the testing results with the compounds of the invention. For some of the compounds, the $MIC_{80}$ value is based on the testing of fewer than 15 rhinovirus serotypes. In these cases the number of serotypes (N) is indicated in parentheses after the $MIC_{80}$ figure.

TABLE

| Example No. | MIC (Polio 2) | $MIC_{80}$ (N) (Rhinovirus) |
|---|---|---|
| 1(c) | 2.5 | 0.44 |
| 2 | 0.02 | 1.33 |
| 3 | 1.0 | 0.57 |
| 4 | IA | 2.675 (4) |
| 6 | 0.9 | 0.94 |
| 7(b) | IA | 0.94 |
| 9(b) | IA | 25.65 (6) |
| 10(b) | 4.4 | 0.7 |
| 11(b) | IA | 0.605 (14) |
| 12 | IA | 1.14 |
| 13(b) | IA | .07 (1)[(a)] |
| 14 | IA | 1.6 |
| 15(b) | IA | 1.86 (5) |
| 16 | IA | 0.56 (13) |
| 19 | IA | 25.33 (6) |
| 20 | 0.06 | 1.2 |
| 21 c | IA | 0.91 |
| 23 (1st compd.) | 2.9 | 0.225 (4) |
| (2nd compd.) | IA | 0.4 (1) [(a)] |
| IA = Inactive | | |
| (a) = Against HRV-2 only | | |

The antiviral compositions are formulated for use by preparing a dilute solution or suspension in a pharmaceutically acceptable aqueous, organic or aqueous-organic medium for parenteral administration by intravenous or intramuscular injection, or for intranasal or ophthalmic application; or are prepared in tablet, capsule, or aqueous suspension form with conventional excipients for oral administration.

**Claims**

**Claims for the following Contracting States : BE, CH, DE, FR, GB, IT, LI, NL, SE**

**1.** A compound of the formula

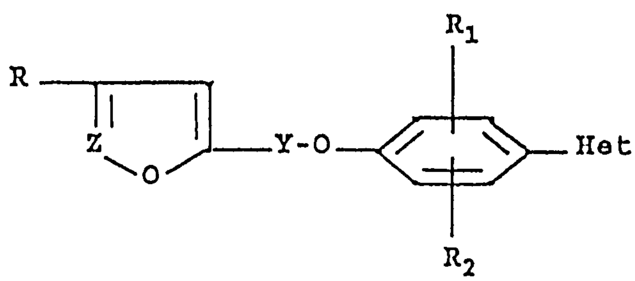

I

wherein:

Y is an alkylene bridge of 3-9 carbon atoms;

Z is N or HC;

R is hydrogen or lower-alkyl of 1-3 carbon atoms, with the proviso that when Z is N, R is lower-alkyl;

$R_1$ and $R_2$ are each hydrogen, halogen, methyl, nitro, lower-alkoxycarbonyl of 2-4 carbon atoms or trifluoromethyl; and

Het is

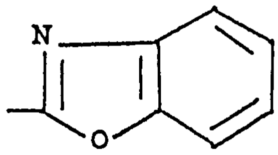 , 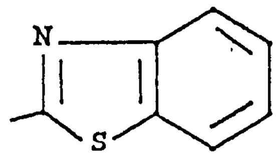 , 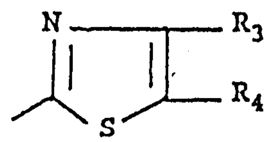 ,

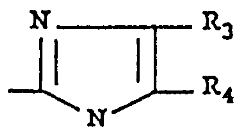 ,

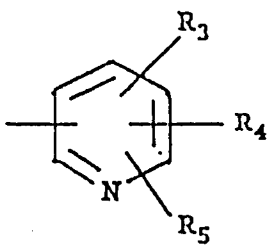 , 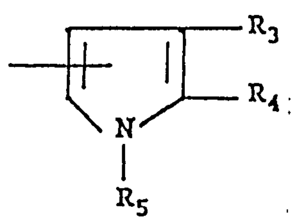 , 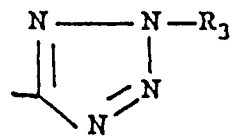 , 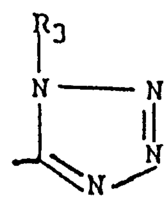 ,

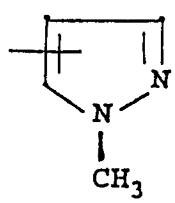 , 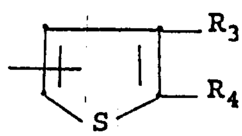

$R_3$, $R_4$ and $R_5$ are hydrogen or lower-alkyl of 1-3 carbon atoms;
or a pharmaceutically acceptable acid-addition salt of a basic member thereof.

2. A compound according to claim 1, of the formula

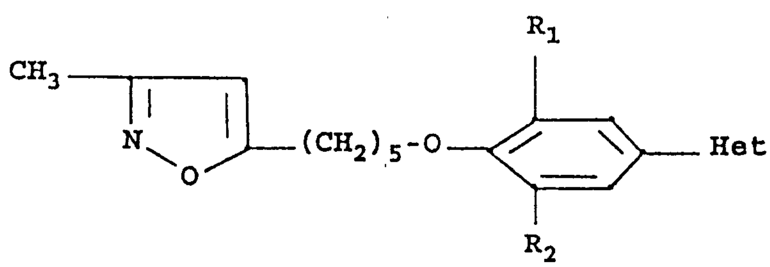

wherein Het, $R_1$ and $R_2$ are as defined in claim 1.

3. A compound according to claim 2, wherein Het is a thienyl or pyridinyl group.

4. A compound according to claim 3, which is 5-{5-[2,6-dichloro-4-(2-thienyl)phenoxy]pentyl}-3-methylisoxazole, or 5-{5-[2,6-dichloro-4-(2-pyridinyl)phenoxy]pentyl}-3-methylisoxazole.

**5.** A process for preparing a compound according to any one of claims 1 to 4, which comprises

(a) reacting a compound of the formula

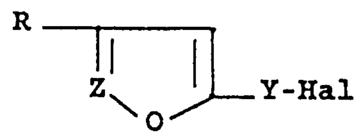

wherein Hal is chlorine, bromine or iodine, and R, Y and Z are as defined in claim 1 with an alkali metal salt of a compound of the formula

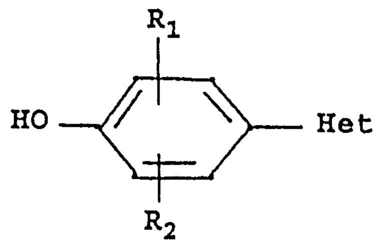

wherein $R_1$, $R_2$ and Het are as defined in claim 1; or

(b) reacting a compound of the formula

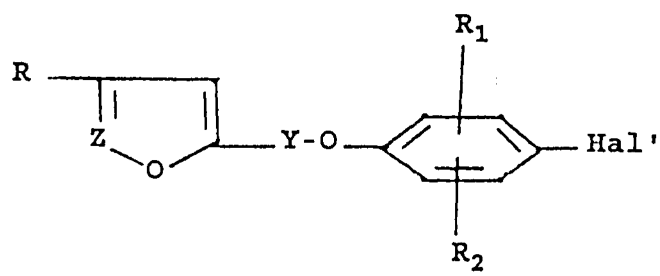

V

where Hal' is bromine or iodine and wherein $R_1$, $R_2$, Y and Z are as defined in claim 1, with a compound of the formula

$(R')_3Sn$-Het

where R' is lower-alkyl of 1-6 carbon atoms, and Het is as defined in claim 1; in the presence of a palladium complex catalyst;

(c) to prepare a compound according to claim 1, wherein Het is a 2-thiazolyl group, converting a compound of Formula IX

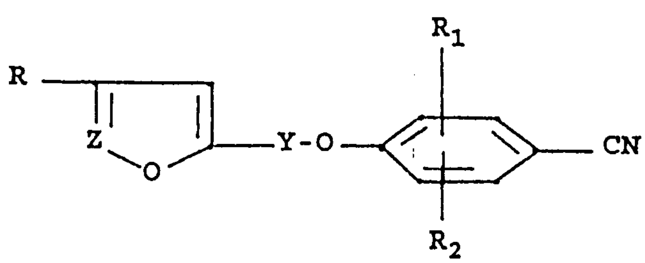

IX

wherein R, $R_1$, $R_2$, Y and Z are as defined in claim 1 to the corresponding thioamide with hydrogen sulfide in pyridine, and then reacting the thioamide with a haloalkanone, $R_3CH(Hal)$-CO-$R_4$ wherein $R_3$ and $R_4$ are as defined in claim 1 and Hal is a halogen atom;

(d) to prepare a compound according to claim 1, wherein Het is a tetrazole group, reacting a compound of Formula IX with sodium azide to give a tetrazole wherein $R_3$ is hydrogen, and, if desired, treating said tetrazole with a lower-alkyl halide in the presence of a base to give both isomeric tetrazoles wherein $R_3$ is lower-alkyl;

and, if desired, converting a basic compound obtained to an acid-addition salt thereof.

**6.** A process according to claim 5, wherein in alternative (b) the palladium complex catalyst is dichlorobis-(triphenylphosphine)palladium.

**7.** A composition for combating viruses which comprises an antivirally effective amount of a compound according to any one of claims 1-4, in admixture with a suitable carrier or diluent.

**8.** Use of a compound as defined in claim 1 to prepare a medicament useful as a viricide.

**Claims for the following Contracting State : AT**

**1.** A process for preparing a compound of the formula

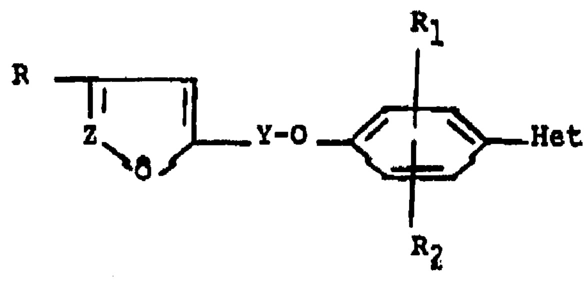

I

wherein:

Y is an alkylene bridge or 3-9 carbon atoms;

Z is N or HC;

R is hydrogen or lower-alkyl of 1-3 carbon atoms, with the proviso that when Z is N, R is lower-alkyl;

$R_1$ and $R_2$ are each hydrogen, halogen, methyl, nitro, lower-alkoxycarbonyl of 2-4 carbon atoms or trifluoromethyl; and

Het is

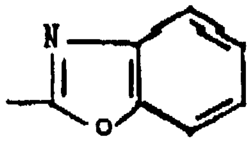 , 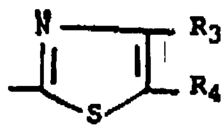 , 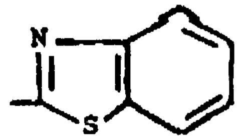 ,

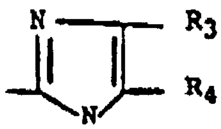 ,

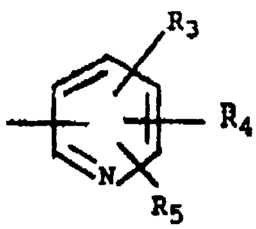 , 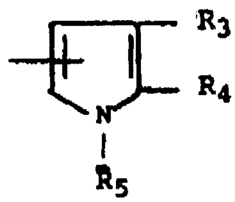 , 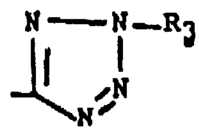 , 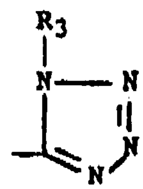 ,

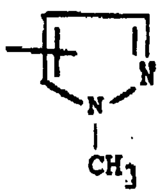 , or 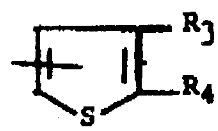

; and

$R_3$, $R_4$ and $R_5$ are hydrogen or lower-alkyl of 1-3 carbon atoms;

or a pharmaceutically acceptable acid-addition salt of a basic member thereof, characterized in that it comprises

(a) reacting a compound of the formula

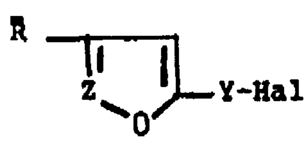

wherein Hal is chlorine, bromine or iodine, with an alkali metal salt of a compound of the formula

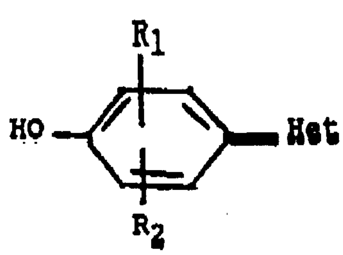 ;

or

(b) reacting a compound of the formula

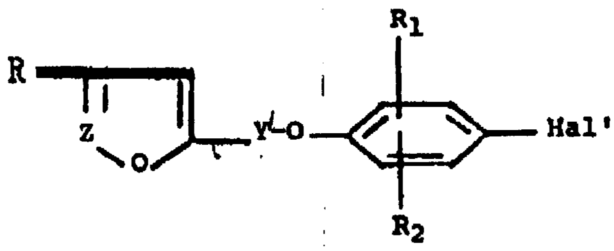

V

where Hal' is bromine or iodine, with a compound of the formula

$(R')_3Sn$-Het'

where R' is lower-alkyl of 1-6 carbon atoms, in the presence of a palladium complex catalyst; or

(c) to prepare a compound of formula I wherein Het is a 2-thiazolyl group, converting a compound of Formula IX

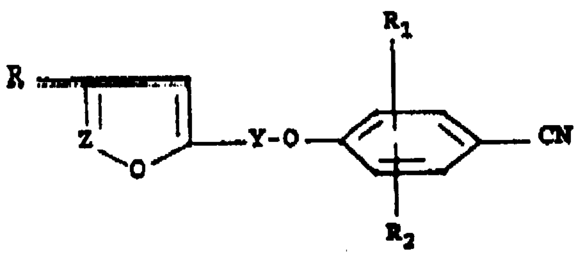

IX

to the corresponding thioamide with hydrogen sulfide in pyridine, and then reacting the thioamide with a haloalkanone, $R_3CH(Hal)-CO-R_4$ wherein Hal is a halogen atom; or

(d) to prepare a compound of formula I wherein Het is a tetrazole group, reacting a compound of Formula IX with sodium azide to give a tetrazole wherein $R_3$ is hydrogen, and, if desired, treating said tetrazole with a lower-alkyl halide in the presence of a base to give both isomeric tetrazoles wherein $R_3$ is lower-alkyl; and, if desired, converting a basic compound obtained to an acid-addition salt thereof.

**2.** A process according to claim 1, wherein in alternative (b) the palladium complex catalyst is dichlorobis-(triphenylphosphine)palladium.

**3.** A process according to claim 1, characterized by producing a product of the formula

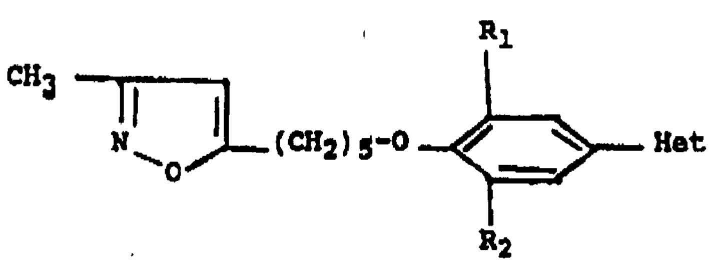

wherein Het, $R_1$ and $R_2$ are as defined in claim 1.

**4.** A process according to any one of the preceding claims, characterized in that Het is a thienyl or pyridinyl group.

**5.** A process according to claim 4, characterized by producing 5-{5-[2,6-dichloro-4-(2-thienyl)phenoxy]-pentyl}-3-methylisoxazole or 5-{5-[2,6-Dichloro-4-(2-pyridinyl)phenoxy]pentyl}-3-methylisoxazole.

**6.** A process according to claim 1, characterized in that $R_1$ and $R_2$ are hydrogen, halogen, methyl, nitro, or trifluoromethyl; and

Het is

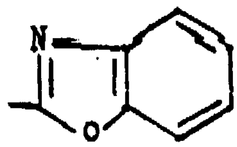 , 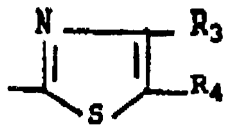 , 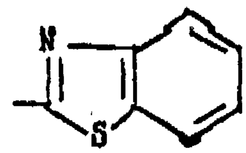 ,

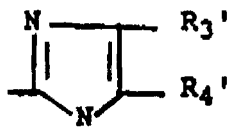 ,

$R_3$ and $R_4$ are hydrogen or lower-alkyl of 1-3 carbon atoms; and

$R_3$' and $R_4$' are hydrogen.

## Patentansprüche

## Patentansprüche für folgende Vertragsstaaten : BE, CH, DE, FR, GB, IT, LI, NL, SE

**1.** Verbindung der Formel

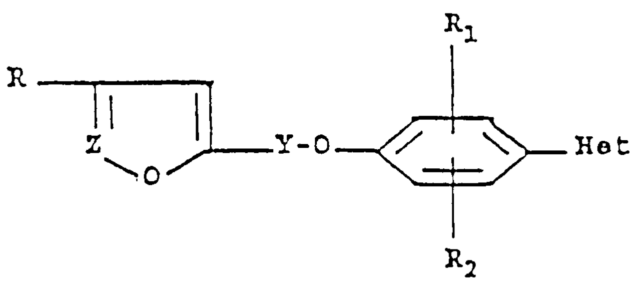

I

worin sind:

Y eine Alkylenbrücke mit 3 bis 9 Kohlenstoffatomen,

Z N oder HC,

R Wasserstoff oder niederes Alkyl mit 1 bis 3 Kohlenstoffatomen unter der Voraussetzung, daß R ein niederes Alkyl ist, wenn Z N ist,

$R_1$ und $R_2$ jeweils Wasserstoff, Halogen, Methyl, Nitro-, niederes Alkoxycarbonyl mit 2 bis 4 Kohlenstoffatomen oder Trifluormethyl, und

Het

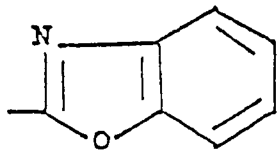 , 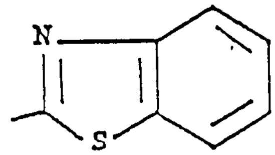 , 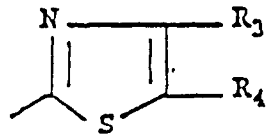 ,

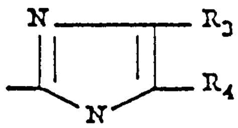 ,

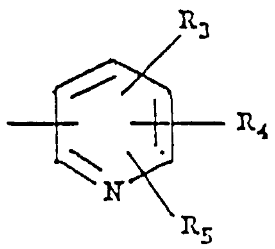 , 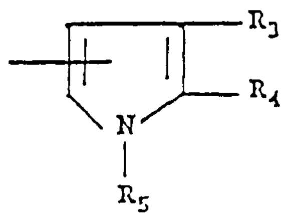 , 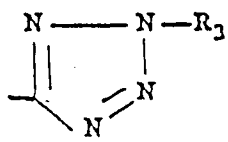 , 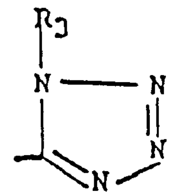 ,

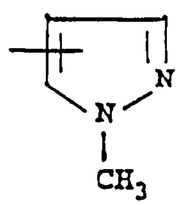 , 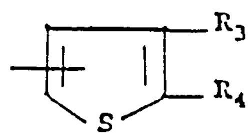

$R_3$, $R_4$ und $R_5$ Wasserstoff oder niederes Alkyl mit 1 bis 3 Kohlenstoffatomen, oder ein pharmazeutisch zulässiges Säure-Anlagerungssalz eines basischen Vertreters von ihnen.

2. Verbindung nach Anspruch 1 der Formel

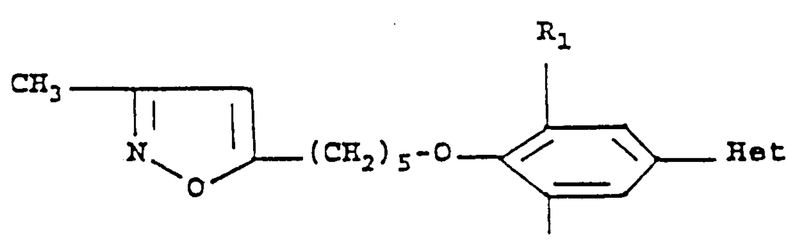

darin sind Het, $R_1$ und $R_2$ wie in Anspruch 1 festgelegt.

3. Verbindung nach Anspruch 2, worin Het eine Thienyl- oder oder Pyridinyl-Gruppe ist.

4. Verbindung nach Anspruch 3, die 5-{5-[2,6-Dichlor-4-(2-thienyl)phenoxy]pentyl}-3-methylisoxazol oder 5-{5-[2,6-Dichlor-4-(2-pyridinyl)phenoxy]pentyl}-3-methylisoxazol ist.

**5.** Verfahren zur Herstellung einer Verbindung nach einem der Ansprüche 1 bis 4, welches umfaßt:
(a) Umsetzen einer Verbindung der Formel

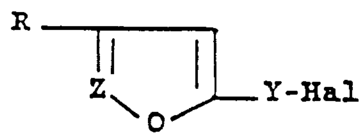

worin Hal Chlor, Brom oder Iod ist und R, Y und Z wie in Anspruch 1 festgelegt, mit einem Alkalimetallsalz einer Verbindung der Formel

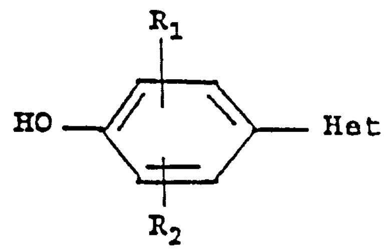

darin sind $R_1$, $R_2$ und Het wie in Anspruch 1 festgelegt, oder
(b) Umsetzen einer Verbindung der Formel

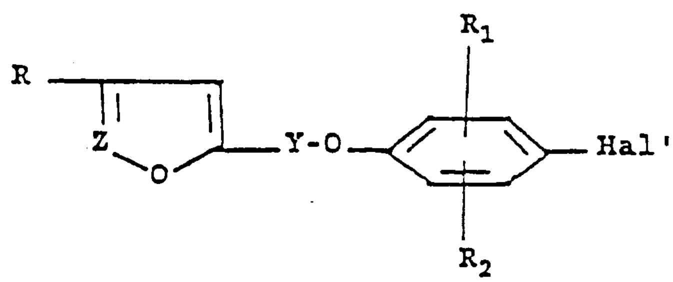

V

darin ist Hal' Brom oder Iod und $R_1$, $R_2$, Y und Z wie in Anspruch 1 festgelegt, mit einer Verbindung der Formel

$(R')_3Sn$-Het

worin R' niederes Alkyl mit 1 bis 6 Kohlenstoffatomen ist und Het wie in Anspruch 1 festgelegt, und zwar in Gegenwart eines Palladiumkomplex-Katalysators;
(c) um eine Verbindung nach Anspruch 1 herzustellen, worin Het eine 2-Thiazolyl-Gruppe ist, Umwandeln einer Verbindung der Formel IX

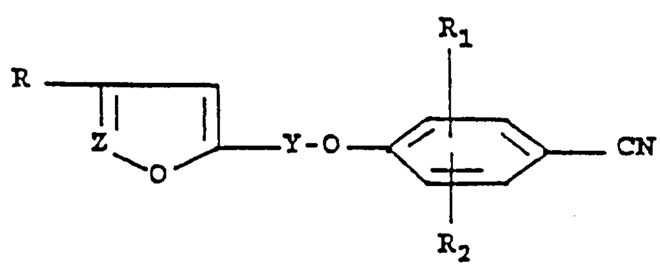

IX

worin R, $R_1$, $R_2$, Y und Z wie in Anspruch 1 festgelegt sind, in das entsprechende Thioamid mit Schwefelwasserstoff in Pyridin, und sodann Umsetzen des Thioamids mit einem Haloalkanon, $R_3$CH-

(Hal)-CO-$R_4$, darin sind $R_3$ und $R_4$ wie in Anspruch 1 festgelegt und Hal ein Halogenatom;
(d) um eine Verbindung nach Anspruch 1 herzustellen, worin Het eine Tetrazol-Gruppe ist, Umsetzen einer Verbindung der Formel IX mit Natriumazid, um ein Tetrazol zu ergeben, worin $R_3$ Wasserstoff ist und, wenn angestrebt, Behandeln des Tetrazols mit einem niederen Alkylhalogenid in Anwesenheit einer Base, um beide isomere Tetrazole zu ergeben, worin $R_3$ niederes Alkyl ist, und, wenn angestrebt, Umwandeln einer erhaltenen basischen Verbindung zu ihrem Säure-Anlagerungssalz.

**6.** Verfahren nach Anspruch 5, bei welchem alternativ (b) der Palladiumkomplex-Katalysator Dichlorbis-(triphenylphosphin)palladium ist.

**7.** Zusammensetzung zur Virusbekämpfung, die eine antiviral wirksame Menge einer Verbindung nach einem der Ansprüche 1 bis 4 in Zumischung mit einem geeigneten Träger oder Streckmittel aufweist.

**8.** Verwendung einer Verbindung wie unter Anspruch 1 festgelegt wurde, um ein als ein Viricid verwendbares Medikament herzustellen.

**Patentansprüche für folgenden Vertragsstaat : AT**

**1.** Verfahren zum Herstellen einer Verbindung der Formel

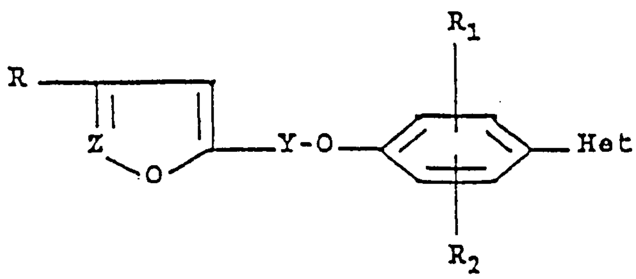

I

worin sind:
Y eine Alkylenbrücke mit 3 bis 9 Kohlenstoffatomen,
Z N oder HC,
R Wasserstoff oder niederes Alkyl mit 1 bis 3 Kohlenstoffatomen

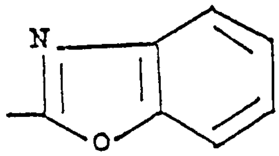 , 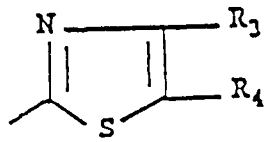 , 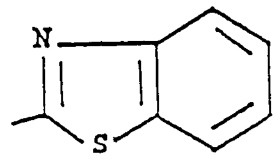 ,

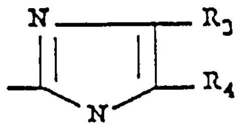 ,

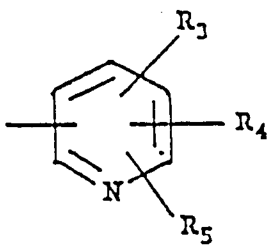 , 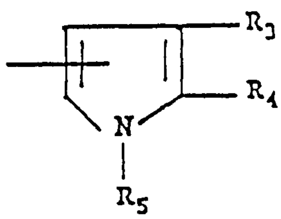 , 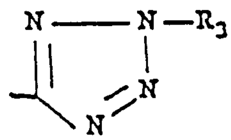 , 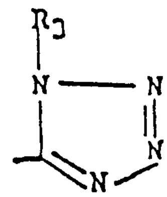 ,

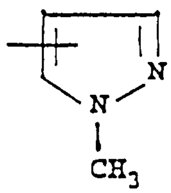 oder 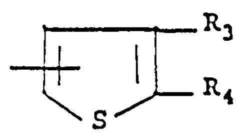 ;

und $R_3$, $R_4$ und $R_5$ Wasserstoff oder niederes Alkyl mit 1 bis 3 Kohlenstoffatomen; oder ein pharmazeutisch zulässiges Säure-Anlagerungssalz eines basischen Vertreters von ihnen, dadurch gekennzeichnet, daß es umfaßt:

(a) Umsetzen einer Verbindung der Formel

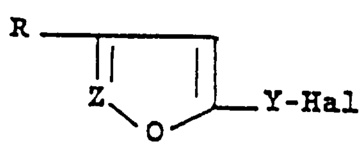

worin Hal Chlor, Brom oder Iod ist, mit einem Alkalimetallsalz einer Verbindung der Formel

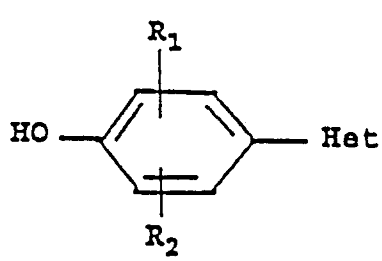

oder

(b) Umsetzen einer Verbindung der Formel

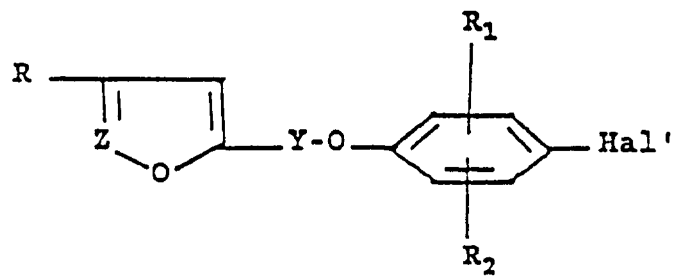

V

worin Hal' Brom oder Iod ist, mit einer Verbindung der Formel

$(R')_3Sn$-Het'

worin R' niederes Alkyl mit 1 bis 6 Kohlenstoffatomen ist, in Anwesenheit eine Palladiumkomplex-Katalysators, oder

(c) um eine Verbindung der Formel I herzustellen, worin Het eine 2-Thiazolyl-Gruppe ist, Umsetzen einer Verbindung der Formel IX

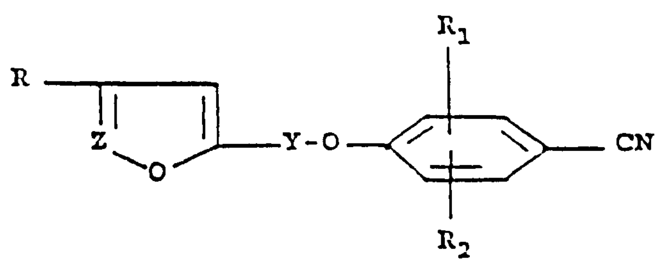

IX

zu dem entsprechenden Thioamid mit Schwefelwasserstoff in Pyridin, und sodann Umsetzen des Thioamids mit einem Haloalkanon, $R_3CH(Hal)$-CO-$R_4$, worin Hal ein Halogenatom ist, oder

(d) um eine Verbindung der Formel I herzustellen, worin Het eine Tetrazol-Gruppe ist, Umsetzen einer Verbindung der Formel IX mit Natriumazid, um ein Tetrazol zu ergeben, worin $R_3$ Wasserstoff ist und, wenn angestrebt, Behandeln des Tetrazols mit einem niederen Alkylhalogenid in Anwesenheit einer Base, um beide isomere Tetrazole zu ergeben, worin $R_3$ niederes Alkyl ist und, wenn angestrebt, Umwandeln einer erhaltenen basischen Verbindung in ihr Säure-Anlagerungssalz.

**2.** Verfahren nach Anspruch 1, bei welchem alternativ (b) der Palladiumkomplex-Katalysator Dichlorbis-(triphenylphosphin)palladium ist.

**3.** Verfahren nach Anspruch 1, gekennzeichnet durch das Herstellen eines Produkts der Formel

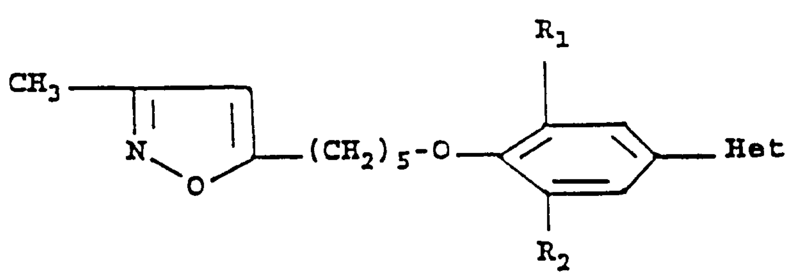

worin sind Het, $R_1$ und $R_2$ wie in Anspruch 1 festgelegt.

4. Verfahren nach einem dar vorgenannten Ansprüche, dadurch gekennzeichnet, daß Hat eine Thienyl- oder Pyridinyl-Gruppe ist.

5. Verfahren nach Anspruch 4, gekennzeichnet durch die Herstellung von 5-{5-[2,6-Dichlor-4-(2-thienyl)-phenoxy]pentyl}-3-methylisoxazol oder 5-{5-[2,6-Dichlor-4-(2-pyridinyl)phenoxy]pentyl}-3-methylisoxazol.

6. Verfahren nach Anspruch 1, dadurch gekennzeichnet, daß $R_1$ und $R_2$ Wasserstoff, Halogen, Methyl, Nitro- oder Trifluormethyl sind und Het

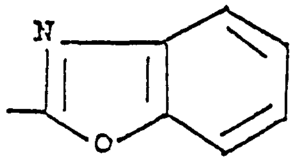 , 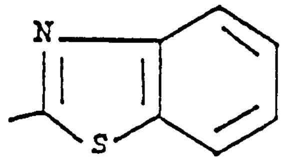 , 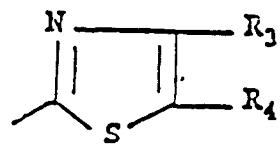 ,

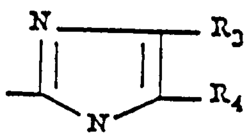 ,

ist,

$R_3$ und $R_4$ Wasserstoff oder niederes Alkyl mit 1 bis 3 Kohlenstoffatomen sind und
$R_3'$ und $R_4'$ Wasserstoff sind.

**Revendications**

**Revendications pour les Etats contractants suivants : BE, CH, DE FR, GB, IT, LI, NL, SE**

1. Composé de formule :

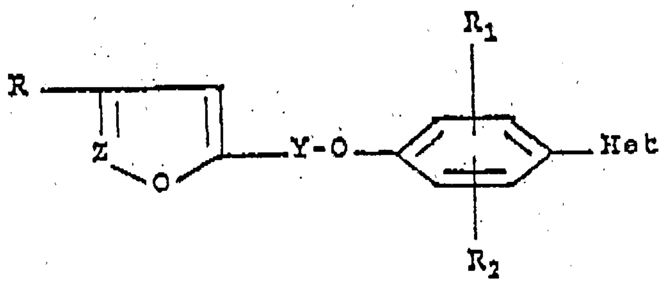

I

dans laquelle :
Y représente un pont alkylène en $C_3$-$C_9$ ;
Z représente N ou HC ;
R représente un atome d'hydrogène ou un groupement alkyle inférieur en $C_1$-$C_3$, à condition que, quand Z représente N, R représente un groupement alkyle inférieur ;
$R_1$ et $R_2$ sont chacun un atome d'hydrogène, un atome d'halogène, un groupement méthyle, un groupement nitro, un groupement alcoxycarbonyle inférieur en $C_2$-$C_4$ ou un groupement trifluorométhyle ; et
Het représente

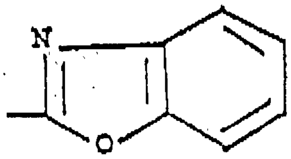 , 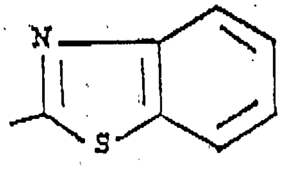 , 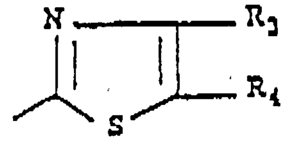 ,

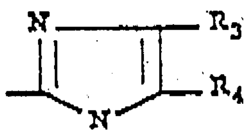 ,

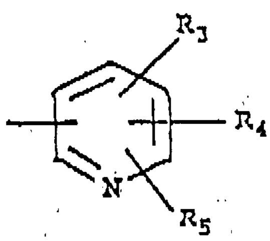 , 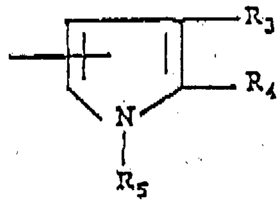 , 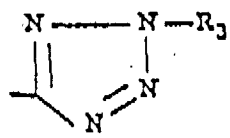 , 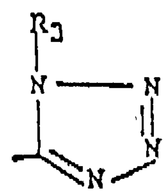 ,

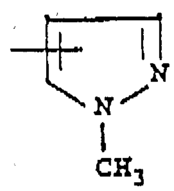 , 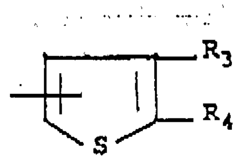

$R_3$, $R_4$ et $R_5$ représentent des atomes d'hydrogène ou des groupements alkyle inférieurs en $C_1$-$C_3$ ; ou un sel d'addition d'acide pharmaceutiquement acceptable d'un élément basique de ceux-ci.

2. Composé selon la revendication 1, répondant à la formule :

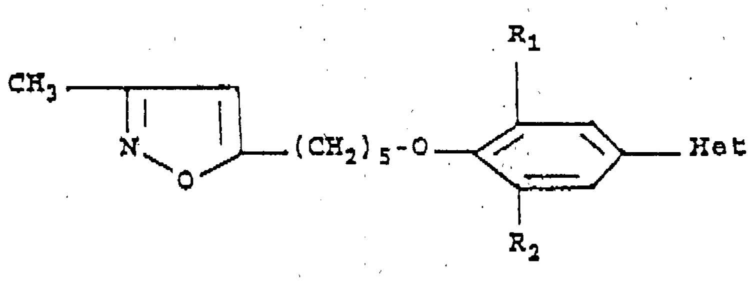

dans laquelle
Het, $R_1$ et $R_2$ sont tels que définis dans la revendication 1.

3. Composé selon la revendication 2, dans lequel Het est un groupement thiényle ou pyridinyle.

4. Composé selon la revendication 3, dans lequel Het est le 5-{5-[2,6-dichloro-4-(2-thiényl)phénoxy]-pentyl}-3-méthylisoxazole, ou le 5-{5-[2,6-dichloro-4-(2-pyridinyl)phénoxy]pentyl}-3-méthylisoxazole.

**5.** Procédé de préparation d'un composé selon l'une quelconque des revendications 1 à 4, qui comprend les étapes consistant à :

a) faire réagir un composé de formule :

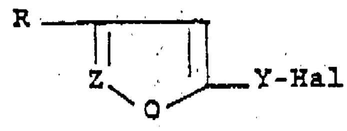

dans laquelle

Hal représente un atome de chlore, de brome ou d'iode et R, Y et Z sont tels que définis dans la revendication 1, avec un sel de métal alcalin d'un composé de formule :

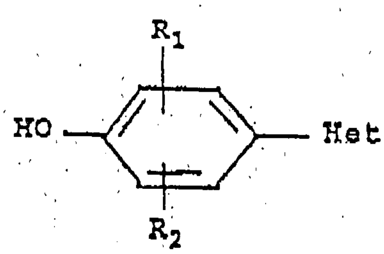

dans laquelle

$R_1$, $R_2$ et Het sont tels que définis dans la revendication 1 ; ou

b) faire réagir un composé de formule :

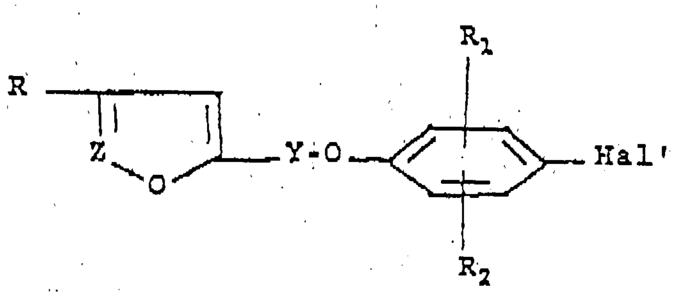

V

dans laquelle

Hal' est un atome de brome ou d'iode et dans laquelle $R_1$, $R_2$, Y et Z sont tels que définis dans la revendication 1, avec un composé de formule :

$(R')_3Sn$-Het

dans laquelle

R' représente un groupement alkyle inférieur en $C_1$-$C_6$ et Het est tel que défini dans la revendication 1 ; en présence d'un catalyseur qui est un complexe du palladium ;

(c) préparer un composé selon la revendication 1, dans lequel Het est un groupement 2-thiazolyle, en transformant un composé de formule IX :

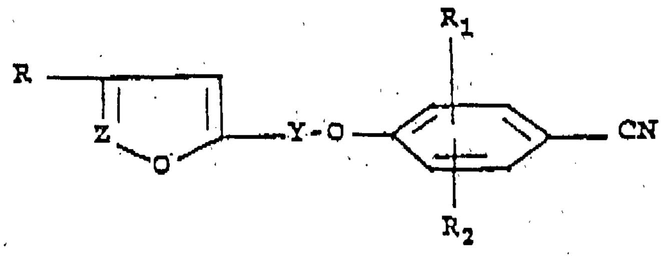

IX

dans laquelle
R, $R_1$, $R_2$, Y et Z sont tels que définis dans la revendication 1, en le thioamide correspondant, avec du sulfure d'hydrogène dans de la pyridine et ensuite en faisant réagir le thioamide avec une halogénoalcanone, $R_3CH(Hal)$-CO-$R_4$ dans laquelle $R_3$ et $R_4$ sont tels que définis dans la revendication 1 et Hal est un atome d'halogène ;
(d) préparer un composé selon la revendication 1, dans lequel Het est un groupement tétrazole, en faisant réagir un composé de formule IX avec un azide de sodium pour donner un tétrazole dans lequel $R_3$ représente un atome d'hydrogène et, si on le souhaite, en traitant ledit tétrazole avec un halogénure d'alkyle inférieur en présence d'une base pour donner deux tétrazoles isomères dans lesquels $R_3$ représente un groupement alkyle inférieur et, si on le souhaite, transformer un composé basique obtenu en un sel d'addition d'acide de celui-ci.

**6.** Procédé selon la revendication 5, dans lequels dans l'alternative (b), le catalyseur qui est un composé du palladium est le dichlorobis(triphénylphosphine)palladium.

**7.** Composition déstinée à combattre des virus qui comprend une quantité antivirale efficace d'un composé selon l'une quelconque des revendications 1 à 4, mélangé avec un véhicule ou un diluant adapté.

**8.** Utilisation d'un composé tel que défini dans la revendication 1, pour préparer un médicament utile en tant que virocide.

**Revendications pour l'Etat contractant suivant : AT**

**1.** Procédé de préparation d'un composé de formule :

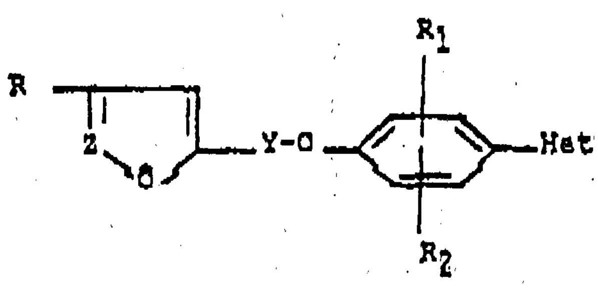

I

dans laquelle :
Y représente un pont alkylène en $C_3$-$C_9$ ;
Z représente N ou HC ;
R représente un atome d'hydrogène ou un groupement alkyle inférieur en $C_1$-$C_3$, à condition que, quand Z représente N, R représente un groupement alkyle inférieur ;
$R_1$ et $R_2$ représentent chacun un atome d'hydrogène, un atome d'halogène, un groupement méthyle, un groupement nitro, un groupement alcoxycarbonyle inférieur en $C_2$-$C_4$ ou un groupement trifluorométhyle ; et
Het représente

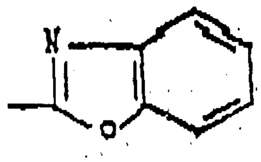 , 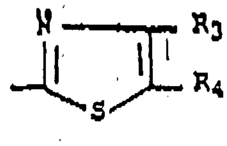 , 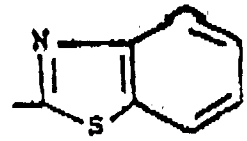 ,

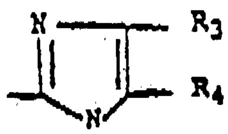 ,

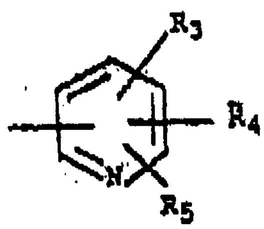 , 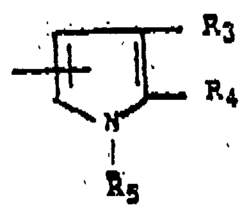 , 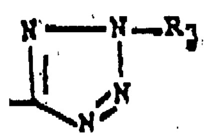 , 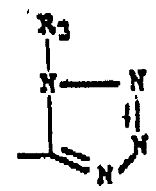 ,

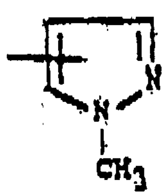 , ou 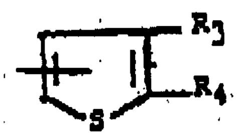

et $R_3$, $R_4$ et $R_5$ représentent des atomes d'hydrogène ou des groupements alkyle inférieurs en $C_1$-$C_3$ ; ou un sel d'addition d'acide pharmaceutiquement acceptable d'un élément basique de ceux-ci, caractérisé on ce qu'il comprend les étapes consistant à :

a) faire réagir un composé de formule :

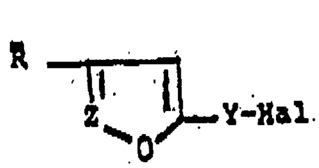

dans laquelle

Hal représente un atome de chlore, de brome ou d'iode avec un sel de métal alcalin d'un composé de formule :

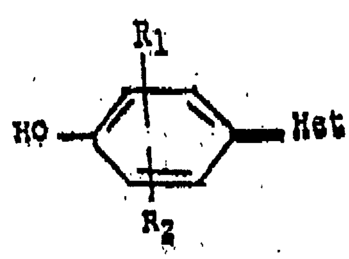 ;

ou

b) faire réagir un composé de formule :

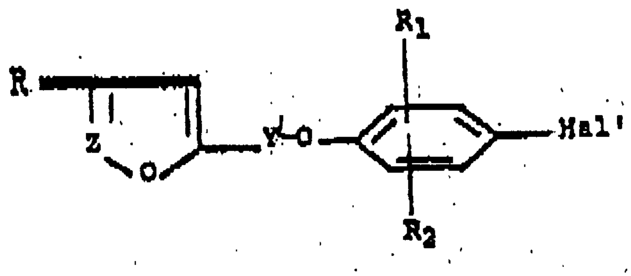

V

dans laquelle

Hal' représente un atome de brome ou un atome d'iode, avec un composé de formule :

$(R')_3Sn$-Het

dans laquelle

R' représente un groupement alkyle inférieur en $C_1$-$C_6$, en présence d'un catalyseur qui est un complexe du palladium ; ou

(c) préparer un composé de formule I dans laquelle Het représente un groupement 2-thiazolyle, en transformant un composé de formule IX :

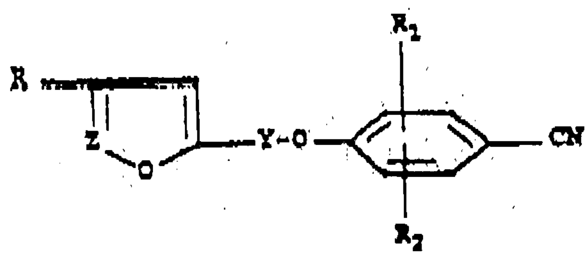

IX

en le thioamide correspondant, en présence de sulfure d'hydrogène dans de la pyridine et ensuite en faisant réagir le thioamide avec une halogénoalcanone, $R_3CH(Hal)$-CO-$R_4$ dans laquelle Hal représente un atome d'halogène ; ou

(d) préparer un composé de formule I, dans lequel Het représente un groupement tétrazole, en faisant réagir un composé de formule IX avec un azide de sodium pour donner un tétrazole dans lequel $R_3$ représente un atome d'hydrogène et si on le souhaite en traitant en ledit tétrazole avec un halogénure d'alkyle inférieur en présence d'une base pour donner les deux tétrazoles isomères dans lesquels $R_3$ représente un groupement alkyle inférieur et, si on le souhaite, en transformant un composé basique obtenu en un sel d'addition d'acide de celui-ci.

2. Procédé selon la revendication 1, dans lequel, dans l'alternative (b), le catalyseur qui est un complexe du palladium est le dichlorobis(triphénylphosphine)palladium.

3. Procédé selon la revendication 1, caractérisé par la production d'un composé de formule :

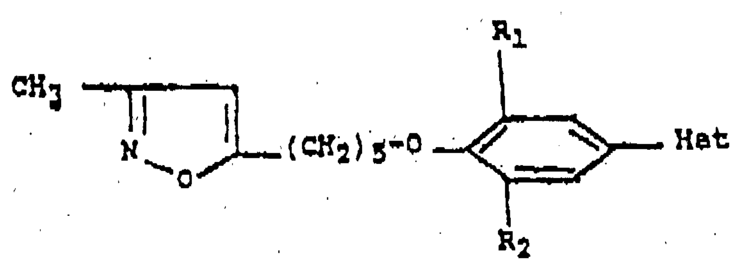

dans laquelle Het, $R_1$ et $R_2$ sont tels que définis dans la revendication 1.

**4.** Procédé selon l'une quelconque des revendications précédentes, caractérisé en ce que Het est un groupement thiényle ou pyridinyle.

**5.** Procédé selon la revendication 4, caractérisé par la production de 5-{5-[2,6-dichloro-4-(2-thiényl)-phénoxy]pentyl}-3-méthylisoxazoleoude5-{5-[2,6-dichloro-4-(2-pyridinyl)phénoxy]pentyl}-3-méthylisoxazole.

**6.** Procédé selon la revendication 1, caractérisé en ce que $R_1$ et $R_2$ représentent des atomes d'hydrogène, d'halogène, des groupements méthyle, nitro ou trifluorométhyle ; et Het représente

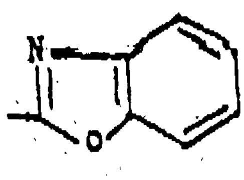 , 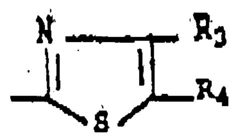 , 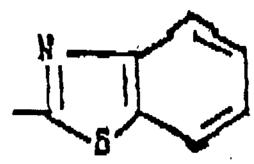 ,

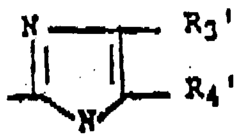 ,

$R_3$ et $R_4$ représentent des atomes d'hydrogène ou des groupements alkyle inférieurs en $C_1$-$C_3$ ; et $R_3$' et $R_4$' représentent des atomes d'hydrogène.